# EUROPEAN PATENT SPECIFICATION

(11) **EP 1 843 741 B1**
(45) Date of publication and mention of the grant of the patent: **03.04.2013**
(21) Application number: 05775135.6
(22) Date of filing: 13.07.2005
(51) Int. Cl.: A61Q 11/00, A61K 8/44, A61K 8/19

(54) **Opaque toothpaste composition**
Opake Zahnpastenzusammensetzung
Composition dentifrice opaque

(30) Priority: 03.08.2004 EP 04254662; 03.08.2004 EP 04254661; 15.11.2004 EP 04257064; 15.11.2004 EP 04257063; 13.01.2005 EP 05250131; 26.01.2005 EP 05250642
(43) Date of publication of application: 17.10.2007
(73) Proprietor: Unilever N.V., 3013 AL Rotterdam (NL); Unilever PLC, London EC4Y 0DY (GB)
(72) Inventor: WATERFIELD, P. C., Unilever R & D Port Sunlight, Bebington merseyside CH63 3 JW (GB)
(74) Representative: James, Helen Sarah
(86) International application number: PCT/EP2005/007718
(87) International publication number: WO 2006/012987

(56) References cited:
- EP-A- 0 740 932
- WO-A-00/38644
- GB-A- 1 319 247
- US-A- 4 325 939
- US-A- 4 425 325
- US-A- 4 758 439
- US-A- 5 188 820
- US-A1- 2003 072 721

## Description

The present invention relates to an opaque toothpaste composition comprising bioactive zinc salts in a chalk formulation. The invention also relates to a method for making said composition.

EP-A1-0 740 932 (Unilever) discloses a visually-clear gel type dentifrice comprising a zinc salt which is more water soluble than zinc citrate, an amino acid which can bind zinc and a low refractive index type abrasive silica.

US 5 470 561 (Klugkist) discloses an anti-plaque mouthwash comprising a zinc salt and triclosan. The composition may also comprise glycine and has a pH of between 4 and 8, preferably between 5 and 7, the preferred pH being 6.

GBA-2 052 978 (Unilever) discloses a toothpaste comprising zinc salts with glycine and a pH of from 4.5 to 8.0.

US 5 632 972 (Williams) discloses a method for minimising damage to gingival and periodontal tissue by delivering a first component comprising zinc and a second component comprising a bicarbonate.

US 4 425 325 (Sapone et al) discloses an oral composition containing biologically active zinc ions with glycine and having an adjusted pH of about 4.5 to 9.4.

GB 1 319 247 (Beecham INC) discloses a non-liquid anticalculus dental composition comprising a dental vehicle, and a zinc, cupric or zirconium complex of a fluorinated β-diketone.

US 4 325 939 (Shah) discloses an alkali metal or ammonium zinc citrate prepared for use in dental compositions and especially in mouthwash compositions.

US 5 188 820 (Cummins et al) discloses oral compositions such as dentifrices comprising a mixture of a stannous salt such as stannous fluoride or stannous pyrophosphate and a zinc salt such as zinc citrate.

US 2003/072721 A1 describes toothpaste comprising calcium carbonate and water and also envisages the presence of zinc salts, such as zinc sulphate, as antimicrobial agents.

From WO 00/38644 A it is generally known that the introduction of zinc ions into formulations containing water and bicarbonate and/or carbonate ions leads to the (undesirable) generation of carbon dioxide.

Nevertheless, there has been no attempt in the prior art to produce a toothpaste composition comprising a zinc salt and calcium carbonate as an abrasive. This is due to the undesirable reaction which occurs between the calcium carbonate and the zinc ions to form carbon dioxide. The gas produced in this reaction inflates the closed container in which the composition is stored and will eventually lead to bursting when stored for extended periods of time, for example, between manufacture and use. Furthermore, the production of carbon dioxide reduces the bioavailability of the zinc ions to act as anti-microbial agents, thereby reducing the efficiency of the zinc salts.

In a first aspect of the present invention there is provided an opaque toothpaste composition comprising a zinc salt selected from zinc chloride, zinc sulphate, and zinc fluoride, water, a chelating agent for the zinc salt, the chelating agent having a log Kₛ1 as herein defined of from 3.0 to 7.0, and a calcium carbonate based abrasive present at from 10% to 70% by weight of the toothpaste composition, said abrasive system comprising at least 50% by weight calcium carbonate, and characterised in that the chelating agent is glycine.

The present invention provide an optimum balance between making enough zinc ions bioavailable and capable of interacting with bacteria without forming deleterious amounts of carbon dioxide. This reduces the risk of any gassing of the container in which the composition is stored and, therefore, allows the zinc salt to be stable within the calcium carbonate composition for extended periods of time, for example during storage.

The zinc salt is present at from 0.01 to 5% by weight of the toothpaste composition, preferably from 0.5 to 3.0% by weight of the composition. The zinc salts are selected from zinc chloride, zinc sulphate and zinc fluoride. The preferred zinc salt is zinc sulphate which, in this type of formulation, provides the optimum balance as described above.

It is preferred that the chelating agent for the zinc salt has a log Kₛ1 of from 4.0 to 6.0. The log Kₛ1 is the logarithm of the primary Stability Constant which is the binding affinity for a particular ligand with a particular metal ion, in the present invention zinc. For example, when the chelating agent is glycine, the primary Stability Constant is the binding affinity for one glycine ligand with the free zinc ion and is defined by the following: Kₛ1= [ML] / [M] . [L], where [ML] is the concentration of the metal-ligand complex, [M] is the concentration of the free metal ion and [L] is the concentration of the free ligand. Since [L] is a function of the solution pH, due to the required initial deprotonation of the ligand, as a reference point, the log Kₛ1 values described were recorded at pH 7.4.

The chelating agent is present at from 0.001 to 6% by weight of the composition, preferably at from 0.5 to 4.0% by weight of the composition. Glycine provides an optimal balance between being able to protect the zinc salt from hydrolysis (leading to the formation of carbon dioxide) in the chalk system and keeping it soluble and bioavailable.

The abrasive system is present at from 10 to 70% by weight of the toothpaste composition, preferably at from 20 to 60% and most preferably at from 35 to 45% by weight of the toothpaste composition.

The abrasive system employed in the present invention is calcium carbonate based. This does not prevent the use of non-calcium carbonate abrasives in addition, such as silicas, tungsten carbide and silicon carbide.

The abrasive system comprises at least 50% by weight calcium carbonate, more preferably at least 75% by weight calcium carbonate and most preferably at least 95% by weight calcium carbonate. In the most preferred embodiment of the present invention the abrasive system comprises 100% by weight calcium carbonate although mixtures of calcium carbonate and additional abrasives are possible.

Preferred calcium carbonates include fine ground natural chalk since it has a surprising stability with regard to its interactivity with zinc salts. The term fine ground natural chalk (FGNC) is a known term in the art and suitable examples of such are disclosed in US 2003/0072721 A1 (Riley) the contents of which with regards to the definition, types and grades of FGNC are incorporated herein by reference. Nevertheless, by FGNC is meant chalk which is obtained by milling limestone or marble deposits. Preferably, the FGNC comprises particulate matter of weight-based median particle size ranging from 1 to 15 µm and BET surface area ranging from 0.5 to 3 m²/g.

The composition according to the invention also preferably comprises a fluoride ion source such as an alkali metal salt of monofluorophosphate, preferably sodium monofluorophosphate. Such fluoride ion source will be present at such an amount to provide free fluoride ion at from 100 to 2000 ppm, preferably from 900 to 1500 ppm.

The toothpaste composition according to the invention comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the toothpaste composition. Preferred abrasives are chalk and silica, more preferably fine ground natural chalk.
Humectants such as glycerol, sorbitol, propyleneglycol, xylitol, lactitol etc.;
binders and thickeners such as sodium carboxymethylcellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the toothpaste composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

In a second aspect of the invention there is provided a method of making an opaque toothpaste composition according to the first aspect the method comprising the steps of forming a zinc-chelate solution and buffering to a pH which is equal to or higher than that of the calcium carbonate in an aqueous suspension before mixing with the suspended calcium carbonate. Further materials such as flavours may be added to the final mix.

Embodiments according to the invention shall now be discussed with reference to the following non-limiting examples.

### EXAMPLE 1

The following example formulation is an embodiment according to the invention. It is made by mixing first the zinc sulphate heptahydrate with glycine (and other minors) and buffering to a pH of from 8.5 to 9.5 with sodium hydroxide. The calcium carbonate, surfactant and structurants are then added to the zinc-chelate mix. The flavours are added last.

| Ingredient | % (w/w) |
|---|---|
| | |
| Fine Ground Natural Chalk (Addon 1015) | 40.00 |
| Sorbitol (70% aq) | 15.00 |
| Thickening silica | 3.00 |
| Sodium carboxymethyl cellulose | 0.90 |
| Flavour | 1.10 |
| Sweetener | 0.23 |
| Sodium lauryl sulphate | 2.50 |
| Zinc sulphate heptahydrate | 2.745 |
| Glycine | 3.584 |
| Sodium Hydroxide | 1.12 |
| Formalin | 0.10 |
| Sodium monofluorophosphate | 0.76 |
| Water | to 100 |
| | 100.00 |

### EXAMPLE 2

The Table shows the upper aqueous solution pH that can be attained without precipitating zinc hydroxide when the zinc is protected by a 1:3 ratio of zinc to acetic acid or glycine acting as ligands.

| molar ratio of zinc to ligand | acetic acid | glycine |
|---|---|---|
| zinc-ligand (log Kₛ1) | 1.03 | 5.16 |
| pH at which zinc hydroxide precipitates | 5.82 | >10 |

In other words at a 1:3 ratio of zinc to ligand and where acetate is the ligand zinc hydroxide will form when the pH of the composition reaches just below 6. The pH of toothpaste compositions comprising calcium carbonate as abrasive typically reaches as low as 8 with most being much above 8 and even as high as 10.

Accordingly, of the two, only glycine can act as ligand for zinc in a chalk toothpaste since only such a ligand can prevent formation of zinc hydroxide and the resulting carbon dioxide on reaction with calcium in the chalk.

## Claims

1. An opaque toothpaste composition comprising water, a zinc salt selected from zinc chloride, zinc sulphate, and zinc fluoride, a chelating agent for the zinc salt, the chelating agent having a log Kₛ1 of from 3.0 to 7.0, wherein Kₛ1 represents the binding affinity for the chelating agent with zinc ion and is defined as [ML]/[M].[L], where [ML] is the concentration of the zinc-chelating agent complex, [M] is the concentration of the free zinc ion and [L] is the concentration of the free chelating agent, recorded at pH 7.4; and a calcium carbonate based abrasive system present at from 10% to 70% by weight of the toothpaste composition, said abrasive system comprising at least 50% by weight calcium carbonate, and **characterised in that** the chelating agent is glycine.

2. An opaque toothpaste composition according to any preceding claim, wherein the composition has a pH of from 8.0 to 10.0.

3. An opaque toothpaste composition according to any preceding claim, wherein the calcium carbonate is fine ground natural chalk.

4. An opaque toothpaste composition according to any preceding claim, wherein the composition comprises a fluoride ion source.

5. An opaque toothpaste composition according to any preceding claim, wherein the zinc salt is zinc sulphate.

6. An opaque toothpaste according to any preceding claim comprising an agent selected from the group consisting of anti-caries agents, anti-tartar agents, anti-malodour agents, whitening teeth agents, anti-gingivitis agents and mixtures thereof.

7. Method of making an opaque toothpaste composition according to Claim 1, the method comprising forming an aqueous suspension of calcium carbonate, forming a zinc-chelate solution, buffering the zinc-chelate solution to a pH which is equal to or higher than that of the aqueous suspension of calcium carbonate, and then mixing the zinc-chelate solution with the aqueous suspension of calcium carbonate, and optionally adding any further materials at any stage.

## Patentansprüche

1. Opake Zahnpastenzusammensetzung, umfassend Wasser, ein Zinksalz, das aus Zinkchlorid, Zinksulfat und Zinkchlorid ausgewählt ist, einen Chelatbildner für das Zinksalz, wobei der Chelatbildner einen log Kₛ1 von 3,0 bis 7,0 hat, wobei Kₛ1 die Bindungsaffinität für den Chelatbildner mit Zinkion darstellt und als [ML]/[M].[L] definiert ist, worin [ML] die Konzentration des Zink-Chelatbildner-Komplexes ist, [M] die Konzentration des freien Zinkions ist und [L] die Konzentration des freien Chelatbildners ist, aufgezeichnet bei pH 7,4, und ein Calciumcarbonat-basiertes abrasives System, das mit 10 Gew.-% bis 70 Gew.-% der Zahnpastenzusammensetzung vorliegt, wobei das abrasive System wenigstens 50 Gew.-% Calciumcarbonat umfasst, und **dadurch gekennzeichnet, dass** der Chelatbildner Glycin ist.

2. Opake Zahnpastenzusammensetzung gemäß einem vorangehenden Anspruch, wobei die Zusammensetzung einen pH von 8,0 bis 10,0 hat.

3. Opake Zahnpastenzusammensetzung gemäß einem vorangehenden Anspruch, wobei das Calciumcarbonat fein gemahlene natürliche Kreide ist.

4. Opake Zahnpastenzusammensetzung gemäß einem vorangehenden Anspruch, wobei die Zusammensetzung eine Fluoridionen-Quelle umfasst.

5. Opake Zahnpastenzusammensetzung gemäß einem vorangehenden Anspruch, wobei das Zinksalz Zinksulfat ist.

6. Opake Zahnpasta gemäß einem vorangehenden Anspruch, die ein Mittel, ausgewählt aus der Gruppe, bestehend aus Mitteln gegen Karies, Mitteln gegen Zahnstein, Mitteln gegen Mundgeruch, Bleichmitteln für die Zähne, Mitteln gegen Gingivitis und Gemischen davon, umfasst.

7. Verfahren zur Herstellung einer opaken Zahnpastenzusammensetzung gemäß Anspruch 1, wobei das Verfahren Bilden einer wässrigen Suspension von Calciumcarbonat, Bilden einer Zink-Chelat-Lösung, Puffern der Zink-Chelat-Lösung auf einen pH, der gleich oder höher als der der wässrigen Suspension von Calciumcarbonat ist, und danach Mischen der Zink-Chelat-Lösung mit der wässrigen Suspension von Calciumcarbonat und gegebenenfalls Zusetzen weiterer Materialien in einer beliebigen Stufe umfasst.

## Revendications

1. Composition dentifrice opaque comprenant de l'eau, un sel de zinc choisi parmi le chlorure de zinc, le sulfate de zinc et le fluorure de zinc, un agent chélatant pour le sel de zinc, l'agent chélatant ayant un log Kₛ1 de 3,0 à 7,0, Kₛ1 représentant l'affinité de liaison pour l'agent chélateur avec l'ion zinc et étant défini comme [ML]/[M].[L], où [ML] est la concentration du complexe zinc-agent chélatant, [M] est la concentration de l'ion zinc libre et [L] est la concentration de l'agent chéla chélatant teur libre, enregistrées à pH 7,4 ; et un système abrasif à base de carbonate de calcium présent à une valeur de 10 % à 70 % en poids de la composition dentifrice, ledit système abrasif comprenant au moins 50 % en poids de carbonate de calcium, et **caractérisée en ce que** l'agent chélatant est de la glycine.

2. Composition dentifrice opaque selon l'une quelconque des revendications précédentes, la composition ayant un pH de 8,0 à 10,0.

3. Composition dentifrice opaque selon l'une quelconque des revendications précédentes, dans laquelle le carbonate de calcium est de la craie naturelle finement broyée.

4. Composition dentifrice opaque selon l'une quelconque des revendications précédentes, la composition comprenant une source d'ions fluorure.

5. Composition dentifrice opaque selon l'une quelconque des revendications précédentes, dans laquelle le sel de zinc est du sulfate de zinc.

6. Dentifrice opaque selon l'une quelconque des revendications précédentes, comprenant un agent choisi dans le groupe constitué par les agents anti-caries, les agents anti-tartre, les agents anti-mauvaises odeurs, les agents de blanchiment des dents, les agents anti-gingivite et les mélanges de ceux-ci.

7. Méthode de fabrication d'une composition dentifrice opaque selon la revendication 1, la méthode comprenant la formation d'une suspension aqueuse de carbonate de calcium, la formation d'une solution de zinc-chélate, le tamponnage de la solution de zinc-chélate à un pH qui est supérieur ou égal à celui de la suspension aqueuse de carbonate de calcium, et puis le mélange de la solution de zinc-chélate avec la suspension aqueuse de carbonate de calcium, et l'ajout éventuel d'autres substances quelconques à un stade quelconque.
